(19) European Patent Office — Europäisches Patentamt / European Patent Office / Office européen des brevets

(11) **EP 1 536 212 A2**

(12) **EUROPEAN PATENT APPLICATION**

(43) Date of publication:
01.06.2005 Bulletin 2005/22

(51) Int Cl.7: **G01F 1/66**

(21) Application number: 04027962.2

(22) Date of filing: 25.11.2004

(84) Designated Contracting States:
**AT BE BG CH CY CZ DE DK EE ES FI FR GB GR HU IE IS IT LI LU MC NL PL PT RO SE SI SK TR**
Designated Extension States:
**AL HR LT LV MK YU**

(30) Priority: 27.11.2003 JP 2003396755

(71) Applicant: **FUJI ELECTRIC SYSTEMS CO., LTD.**
**Tokyo, 102-0075 (JP)**

(72) Inventors:
• **Hirayama, Noritomo**
  **Chiyoda-ku Tokyo 102-0075 (JP)**
• **Yamamoto, Toshihiro**
  **Chiyoda-ku Tokyo 102-0075 (JP)**
• **Yao, Hironobu**
  **Chiyoda-ku Tokyo 102-0075 (JP)**

(74) Representative: **Hoffmann, Eckart, Dipl.-Ing.**
**Patentanwalt,**
**Bahnhofstrasse 103**
**82166 Gräfelfing (DE)**

(54) **Clamp-on type acoustic doppler current profiler**

(57) A clamp-on type acoustic Doppler current profiler by which ultrasound is transmitted from an ultrasound transducer (51), provided external to a piping, to fluid (54) within the piping to measure the flow rate profile of the fluid (54) by using a characteristic in which the frequency of ultrasound is changed by the Doppler effect when reflected by reflectors in the fluid (54). The ultrasound oscillator (51) is fixed to the wedge (52) at an inclination such that, when the acoustic velocities of the longitudinal wave and the shear wave of the ultrasound propagating in the piping (53) are equal to or higher than the acoustic velocity of the longitudinal wave in the wedge (52), the incidence angle of the ultrasound transmitted from the wedge (52) into the piping (53) is equal to or higher than the critical angle of the longitudinal wave that is determined by the acoustic velocity of the longitudinal wave in the wedge (52) and the acoustic velocity of the longitudinal wave in the piping (53) and is equal to or lower than the critical angle of the shear wave that is determined by the acoustic velocity of the longitudinal wave in the wedge (52) and the acoustic velocity of the shear wave in the piping (53).

## FIG. 1

EP 1 536 212 A2

## Description

The present invention relates to a clamp-on type acoustic Doppler current profiler that transmits ultrasound from an ultrasound transducer provided exterior to a tube body into to-be-measured fluid in the tube body, and uses the Doppler effect to measure the flow rate profile of the fluid in a non-contact manner.

A clamp-on type ultrasound flow meter is a flow meter in which an ultrasound transducer is attached to a part of the outer circumferential face of a tube body (e.g., piping) in order to measure, from the exterior of the tube body, the flow rate of the fluid moving in the tube body. Clamp-on type ultrasound flow meters are mainly classified into a first type that utilizes the difference in propagation time and a second type that utilizes the Doppler effect.

The first type use a method by which ultrasound is reciprocated in the tube body so as to move along a path that crosses, on a slanted line, the fluid moving in the tube body, and then, the difference between the time in which the ultrasound propagates along the outward route and the time in which the ultrasound propagates along the return route is used to measure the flow rate of the fluid.

The second type uses a method in which, based on the assumption that suspended particles and/ or air bubbles included in the fluid move at the same speed as the fluid itself, the movement speed of the suspended particles or the like is used to measure the flow rate of the fluid (in the following the term "suspended particles" is used in a broad sense covering also air bubbles etc). Specifically, this method is based on the phenomenon according to which, when ultrasound is transmitted into the fluid, the frequency of the ultrasound is changed by the Doppler effect in accordance with the movement speed of the suspended particles when the ultrasound is reflected by the suspended particles. Thereafter, the frequency of the reflected ultrasound is detected to measure the movement speed of the suspended particles (i.e., the speed of the fluid), thereby measuring the flow rate profile and/or the flow rate.

A conventional technique for such a Doppler ultrasound flow meter is disclosed, for example, in [1]: JP-A-2000-97742 (Figure 1, Figure 2). Figure 6 shows a schematic structure of this flow meter.

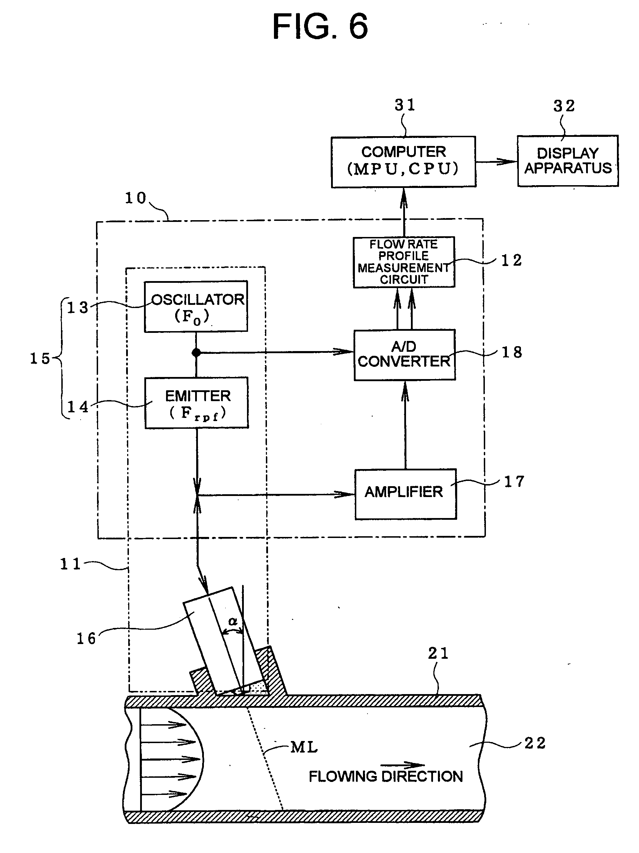

The Doppler ultrasound flow meter shown in Figure 6 includes an ultrasound velocity profile measurement unit (hereinafter referred to as a UVP unit) 10 that measures the flow rate of fluid 22 in a piping 21 in a non-contact manner. This UVP unit 10 includes: an ultrasound transmission means 11 for transmitting to the fluid 22 an ultrasound pulse having a required frequency (basic frequency $f_0$) along a measurement line ML; a flow rate profile measurement circuit 12 for receiving the ultrasound echo reflected from the measurement region of the ultrasound pulse transmitted into the fluid 22 to measure the flow rate profile of the fluid 22 in the measurement region; a computer 31 (e.g., microcomputer, CPU, MPU) for calculating the flow rate profile of the fluid 22 to integrate it in the radial direction of the piping 21, thereby measuring the flow rate of the fluid 22 depending on time; and a display apparatus 32 for displaying the output from this computer 31 in chronological order.

The ultrasound transmission means 11 includes, for example, a signal generator 15 consisting of an oscillator 13 for generating an electric signal having the basic frequency $f_0$ (e.g., 1 MHz, 2 MHz, 4 MHz) and an emitter 14 for outputting the electric signal from this oscillator 13 as periodic pulses having a pulse frequency $F_{rpf}$ and a predetermined cycle of $1/F_{rpf}$, and this signal generator 15 inputs the pulsed electric signal to an ultrasound transducer 16.

The ultrasound transducer 16 transmits the ultrasound pulses having the basic frequency $f_0$ to the fluid 22 in the piping 21 along the measurement line ML. This ultrasound pulse is a straight beam having a beam width of 5 mm for example that has very little dispersion.

The ultrasound transducer 16 is a transmitter/ receiver that is designed also to receive the ultrasound echo generated when a transmitted ultrasound pulse is reflected by reflectors in the fluid 22. The above reflectors are the suspended particles mentioned above that are uniformly included in the fluid 22 (i.e., foreign matter having a different acoustic impedance from that of the fluid 22).

The ultrasound echo received by the ultrasound transducer 16 is converted by this transducer 16 into an electric echo signal. This electric echo signal is amplified by an amplifier 17 in the UVP unit 10 and digitized by an AD converter 18, and then, this digital echo signal is input into the flow rate profile measurement circuit 12.

The electric signal having the basic frequency $f_0$ from the oscillator 13 and digitized by the AD converter 18 is also input to the flow rate profile measurement circuit 12. Based on the difference of frequency (Doppler shift) between these signals, the flow rate is measured to calculate the flow rate profile of the fluid 22 in the measurement region along the measurement line ML. The flow rate profile of this measurement region can be corrected in accordance with the oblique angle $\alpha$ of the ultrasound transducer 16 (oblique angle to the direction perpendicular to the longitudinal direction of the piping 21), thereby measuring the flow rate profile of the fluid 22 in the cross section of the piping 21.

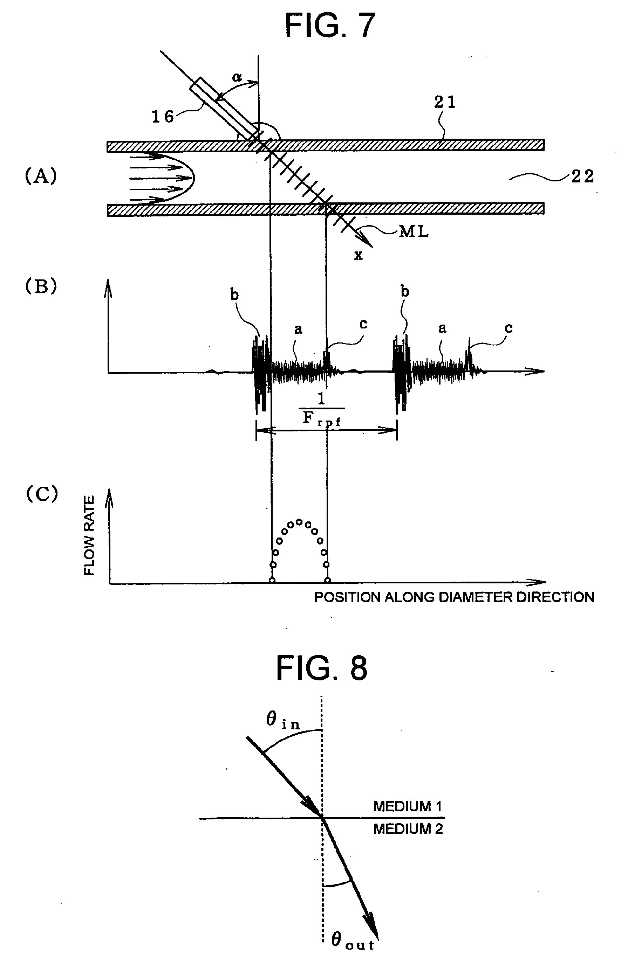

How the Doppler ultrasound flow meter operates will be further described in detail with reference to Figure 7.

As shown in Figure 7(A), the ultrasound transducer 16 is inclined by the oblique angle $\alpha$ to the direction along which the fluid 22 flows, and transmits an ultrasound pulse having the basic frequency $f_0$ into the piping. As a result, this ultrasound pulse collides with and is reflected by the reflectors, i.e., turns into an ultrasound echo "a", which is received by the ultrasound transducer 16, as shown in Figure 7(B).

In Figure 7(B), reference numeral "b" denotes a multiple reflection echo reflected by the tube wall of the piping 21 into which an ultrasound pulse is transmitted, and reference numeral "c" denotes a multiple reflection echo created at the opposing tube wall of the piping 21. The ultrasound transducer 16 transmits ultrasound pulses having a cycle of $(1/F_{rpf})$ as shown in the drawing.

The echo signal "a" received by the ultrasound transducer 16 is filtered and the Doppler shift method is used to measure the flow rate profile along the measurement line ML, thereby providing the display as shown in Figure 7(C). This flow rate profile is measured by the flow rate profile measurement circuit 12 of the UVP unit 10 and is displayed by a display apparatus 32 via the computer 31.

As described above, the Doppler shift method uses the phenomenon according to which, when an ultrasound pulse is transmitted to the fluid 22 flowing in the piping 21, this ultrasound pulse is reflected by reflectors mixed in or uniformly dispersed in the fluid 22 to turn into an ultrasound echo, and the frequency of this ultrasound echo is shifted by a magnitude proportional to the flow rate.

The flow rate profile signal of the fluid 22 measured by the flow rate profile measurement circuit 12 is transmitted to the computer 31 and the flow rate profile signal can be integrated in the radial direction of the piping 21, thereby calculating the flow rate of the fluid 22. The flow rate m(t) of this fluid 22 at time t can be represented by mathematical expression 1:

$$m(t) = \rho \int v\,(x \cdot t) \cdot dA \qquad (1)$$

where

ρ represents the density of the fluid,
v(x·t) represents the velocity component (in direction x) at time t, and
A represents the sectional area of the piping.

The above flow rate m(t) can also be calculated by mathematical expression 2:

$$m(t) = \rho \int \dot{v}x\,(r \cdot \theta \cdot t) \cdot r \cdot dr \cdot d\theta \qquad (2)$$

where
vx(r·θ·t) represents the velocity component at time t from the center on the cross section of the piping in the tube axis direction for distance r and angle θ.

In order to accurately determine the flow rate of the fluid 22 in both the steady state and the non-steady state by the above-described conventional Doppler ultrasound flow meter, the flow rate profile of the fluid 22 in the piping 21 must be detected accurately.

As can be seen from the above-described measurement mechanism, the flow rate profile of the fluid 22 is obtained by subjecting the ultrasound echo to signal processing for calculation. Due to this reason, this ultrasound echo must contain only an acoustic signal, thus requiring the elimination of acoustic noise and electric noise.

Acoustic noise having an influence on this ultrasound echo includes noise caused by the reflection or scattering between the mediums having different acoustic impedances, for example, and noise caused by longitudinal and shear waves generated in solid matter (e.g., the piping material).

Solid matter (e.g., metal) generally includes therein two types of acoustic waves. One is called a compressional wave, a longitudinal wave having a displacement in the same direction as the direction along which the wave propagates, and the other is called a shear wave, a shear wave having a displacement in the direction perpendicular to the direction along which the wave propagates.

According to the document [2]: *"Introduction to electric acoustic engineering"* (SHOKODO Co., Ltd., (P247 to 251)), when an acoustic wave is transmitted from a fluid into a solid matter in an oblique direction, the solid matter includes therein not only a longitudinal wave but also a shear wave. It is generally known that, when an acoustic wave propagates from one type of solid to another type of solid, then both a longitudinal wave and a shear wave are caused along both a direction along which the acoustic wave is transmitted and a direction along which the acoustic wave is reflected.

Hereinafter, how an ultrasound echo is influenced by a longitudinal wave and a shear wave in solid matter will be described.

A case will be described as shown in Figure 8 in which an acoustic wave propagates from medium 1 to medium 2. In this case, the relation between the propagation angle $\theta_{in}$ (incidence angle at the interface between both mediums) and the angle $\theta_{out}$ (refraction angle or output angle at the boundary between both mediums) of the acoustic wave in mediums 1 and 2 can be expressed by mathematical expression 3:

$$\frac{\sin\theta_{in}}{c_1} = \frac{\sin\theta_{out}}{c_2} \qquad (3)$$

where

$c_1$ represents the acoustic velocity in medium 1,
$c_2$ represents the acoustic velocity in medium 2,
$\theta_{in}$ represents the angle in medium 1 (incidence angle), and
$\theta_{out}$ represents the angle in medium 2 (refraction angle).

When the acoustic wave from medium 1 is incident on medium 2 and the acoustic velocity $c_2$ in medium 2 is higher than the acoustic velocity $c_1$ in medium 1 ($c_1 < c_2$), there is a critical angle at which the acoustic wave is totally reflected at the interface between these mediums. This critical angle $\theta c$ is represented by mathematical expression 4:

$$\theta_c = \sin^{-2}(c_1/c_2); (c_1 < c_2) \qquad (4)$$

The following section will describe the oblique angle of the ultrasound transducer 16 of the conventional Doppler ultrasound current profiler shown in Figure 6 (incidence angle of ultrasound to piping 21) in accordance with the following two documents:

[3] *"Development of flow measurement method using ultrasonic velocity profiler (UVP)* (6) *NIST* (*U.S.*) *Calibration: Flow measurement using loops - test results and precision verification"* (Atomic Energy Society of Japan, 1999 autumn convention, 2001)

[4] "*Development of a novel flow metering system using ultrasonic velocity profile measurement",* Experiments in Fluids 32 (2002), 153-160

[3] describes an example in which a so-called clamp-on method is used in which a Doppler ultrasound current profiler is provided at an outer wall of a stainless steel piping for measurement, and in this example, the ultrasound transducer has an oblique angle of 5° or 10°.

[4] describes that an ultrasound transducer driven with a frequency of 1 MHz is provided on the piping with an oblique angle of 5° while an ultrasound transducer driven with a frequency of 4 MHz is provided on the piping at an oblique angle of 0 to 20°, and also describes that the ultrasound transducer and the piping have therebetween an acrylic member having a thickness of 2 mm to be used as a wedge.

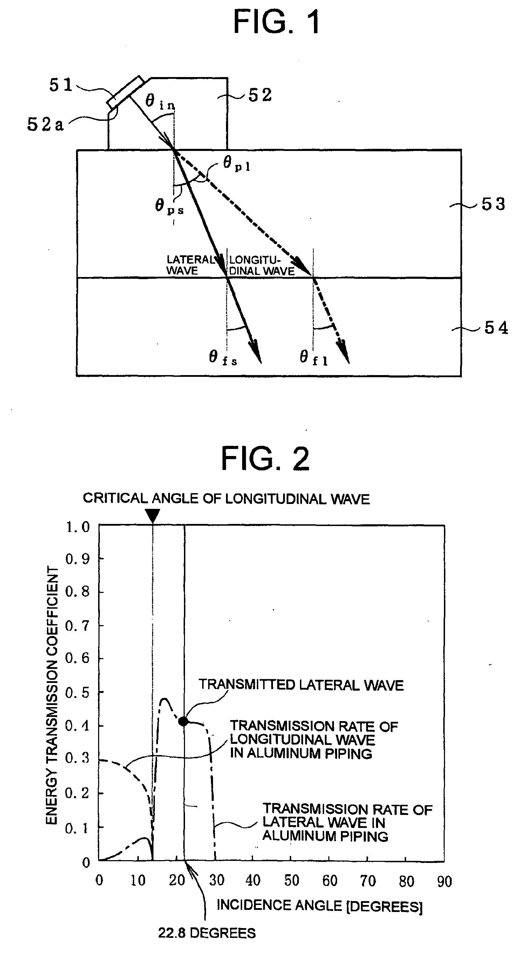

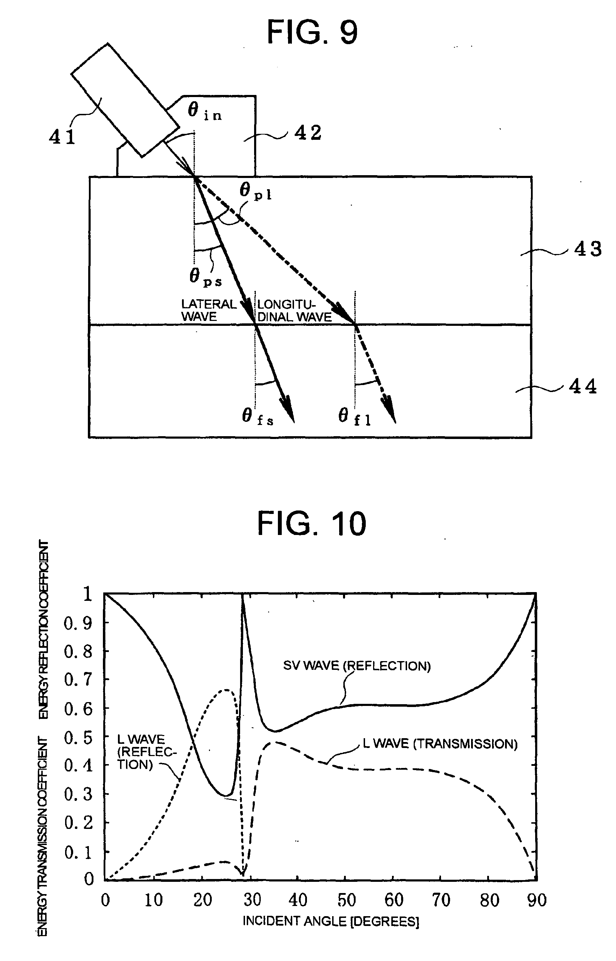

Figure 9 shows the structure in accordance with the measurement conditions described in [4].
In Figure 9, an acrylic wedge 42 is fixed with an ultrasound transducer 41 such that this ultrasound transducer 41 is inclined, with the angle $\theta_{in}$, to a direction perpendicular to the longitudinal direction of a piping 43. Specifically, ultrasound from the wedge 42 to the piping 43 has an incidence angle of $\theta_{in}$.
In [3] and [4], fluid 44 shown in Figure 9 is water while the piping 43 is made of stainless steel. The velocity of sound in water is about 1,500 m/s, the velocity of the longitudinal wave in stainless steel is about 5,750 m/s, and the velocity of the shear wave in stainless steel is about 3,206 m/s. The velocity of the longitudinal wave in acrylic is 2,730 m/s.
The critical angles $\theta_c$ of the longitudinal wave and the shear wave are calculated based on the above-described mathematical expression 4. The critical angle of the longitudinal wave at the interface between the wedge 42 and the piping 43 is 28.3°, and the critical angle of the shear wave at the interface between the wedge 42 and the piping 43 is 58.4°.
When the ultrasound transducer 41 transmits an acoustic wave having an oblique angle (incidence angle) $\theta_{in}$ of 20° for example, the wedge 42 and the piping 43, both of which are solids, have a longitudinal wave and a shear wave at the interface therebetween. The incidence angle $\theta_{in}$ at the above interface is equal to or smaller than the critical angles of both the longitudinal wave and the shear wave. Thus, the piping 43 has therein the propagation of both the longitudinal wave and the shear wave.
Furthermore, the longitudinal wave and the shear wave propagating in the piping 43 are transmitted into water while being refracted. This causes two measurement lines ML.
In the piping 43 shown in Figure 9, the longitudinal wave has a refraction angle (output angle) $\theta_{pl}$ of 46.1° while the shear wave has a refraction angle $\theta_{ps}$ of 23.7°.
When an acoustic wave is transmitted from the piping 43 into water, the acoustic wave is converted to a longitudinal wave and the refraction angle $\theta_{f1}$ in water is 10.84°. The following document [5] discloses the transmission rate of the acoustic wave when, as in the above case, the acoustic wave is transmitted from metal into water.
[5]: "*Ultrasonics Manual"*, Editorial Committee of the Ultrasonics Manual, Maruzen Co., Ltd.
In the example shown in [5], medium 1 is made of aluminum while medium 2 is water in Figure 8.
Figure 10 is a drawing shown in [5] showing the relation, when an aluminum plate corresponding to the medium 1 and water corresponding to the medium 2 have a shear wave at the interface therebetween, between the incidence angle and the energy reflection coefficient (reflectivity) and the energy transmission coefficient (transmission rate). In Figure 10, the wave SV represents a shear wave and the wave L represents a longitudinal wave. As can be seen from Figure 10, total reflection is not caused and the longitudinal wave is transmitted even when the incidence angle of the shear wave exceeds 28°.

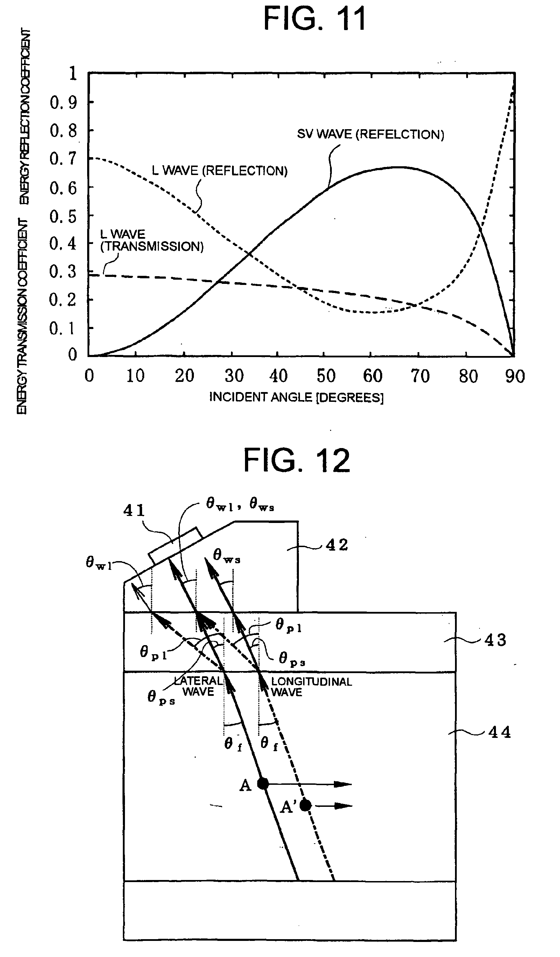

Figure 11 shows the relation, when the aluminum plate and water have a longitudinal wave at the interface therebetween, between the incidence angle and the reflectivity and the transmission rate. As can be seen from Figure 11, only the longitudinal wave is transmitted.
Next, Figure 12 shows the behavior of the ultrasound echo in the structure of Figure 9.
The ultrasound echo from the reflectors in water returns from water to the ultrasound transducer 41 via the same route as that through which ultrasound is transmitted from the piping 43 into water. The ultrasound echo has an incidence angle $\theta_f$ of 10.84° when the ultrasound is transmitted from water into the aluminum piping 43, and thus, both a longitudinal wave and a shear wave are generated, as can be seen from Figure 2 (which will be described later).

As shown in Figure 12, when the ultrasound echo is transmitted from water into the piping 43, two measurement lines of the longitudinal wave and the shear wave are generated, and thus, the piping 43 has therein four ultrasound echoes. In a case that the ultrasound echo is transmitted from the piping 43 into the wedge 42, the acoustic wave has refraction in accordance with mathematical expression 3 but there is no critical angle because the wedge 42 is made of a material having a lower acoustic velocity than that of the piping 43. As a result, no total reflection is caused and four ultrasound echoes progress in the wedge 42 in the direction of the ultrasound transducer 41.

Thus, the four ultrasound echoes propagating in the wedge 42 are transmitted into the ultrasound transducer 41 with a time difference in accordance with the acoustic velocity of the ultrasound transducer 41 in the propagation route.

In Figure 12, $\theta_{pl}$ represents the refraction angle of the longitudinal wave at the interface between the fluid (water) 44 and the piping 43; $\theta_{ps}$ represents the refraction angle of the shear wave; $\theta_{wl}$ represents the refraction angle of the longitudinal wave at the interface between the piping 43 and the wedge 42; and $\theta_{ws}$ represents the refraction angle of the shear wave.

The ultrasound echo received by the ultrasound transducer 41 has a time axis corresponding to the position along the direction of the diameter of the piping 43. The longitudinal wave and the shear wave in the piping 43 have different acoustic velocities.

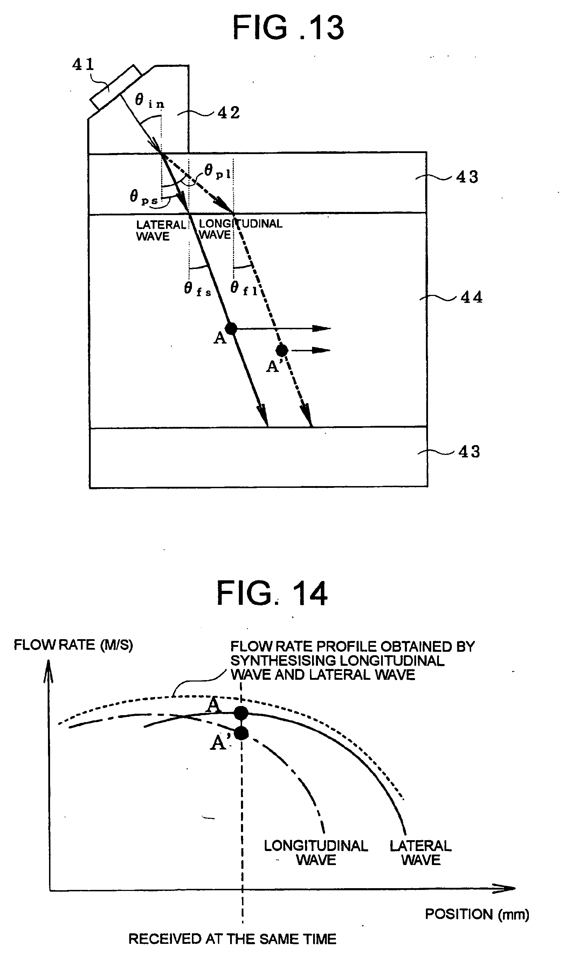

Thus, the ultrasound echo received by the ultrasound transducer 41 at a certain time is obtained by synthesizing the flow rate at point A' of the fluid 44 in the piping 43 measured by the shear wave in Figure 13 with the flow rate at point A' (which is at a different position from that of point A along the direction of the diameter of the piping 43) of the fluid 44 in the piping 43 measured by the longitudinal wave.

Specifically, as schematically shown in Figure 14, the flow rate calculated based on the ultrasound echo received by the ultrasound transducer 41 at a certain time is actually obtained by synthesizing the flow rates at point A and point A' (which are at different positions), and thus, the flow rate profile and consequently the flow rate of the fluid 44 in the piping 43 cannot be measured accurately.

As described above, the Doppler ultrasound flow meter for calculating the flow rate by measuring the flow rate profile in the piping had a problem in that an acoustic wave transmitted from the ultrasound transducer generates a longitudinal wave and a shear wave in a piping that are transmitted along two measurement lines into the fluid, which causes the ultrasound echoes from the respective reflectors received by the Doppler ultrasound flow meter, thus causing the flow rate profile to be measured inaccurately and deteriorating the measurement accuracy.

In view of the above, it is an objective of the present invention to provide a clamp-on type Doppler ultrasound current profiler that can measure a flow rate profile and a flow rate more accurately.

This object is achieved by a clamp-on type Doppler ultrasound current profiler as claimed in claims 1 and 2. Preferred embodiments of the invention are subject-matter of the dependent claims.

According to the invention the object is achieved by eliminating, from the ultrasound echoes caused by a longitudinal wave and a shear wave propagating along two measurement lines in the tube body (e.g., piping), the ultrasound echo caused by the longitudinal wave.

According to the invention of claim 1, the longitudinal wave component of the ultrasound that is transmitted from an ultrasound transducer and that propagates from a wedge to a tube body can be eliminated, thus the fluid has therein only ultrasound along one measurement line, caused by the shear wave in the tube body. As a result, only the ultrasound echo caused by reflection of the shear wave by the reflectors in the fluid appears and thus the ultrasound echo caused by the longitudinal wave is not received by the ultrasound transducer, thus reducing the acoustic noise.

According to the invention of claim 2, the longitudinal wave component of the ultrasound echo from the fluid propagating in the tube body can be eliminated and the ultrasound transducer can receive only the shear wave of the ultrasound echo, thus reducing the acoustic noise as in the case of the invention according to claim 1.

The two embodiments according to claims 1 and 2 can both improve the measurement accuracy of the flow rate profile, which enables determination of the flow rate with a higher accuracy.

Hereinafter, embodiments of the present invention will be described with reference to the drawings.

Figure 1      shows the structure of the main part illustrating the first embodiment of the present invention.

Figure 2      shows the relation between the incidence angle of the ultrasound from water into the piping and the energy transmission coefficients of the longitudinal wave and the shear wave in the piping as known from [6].

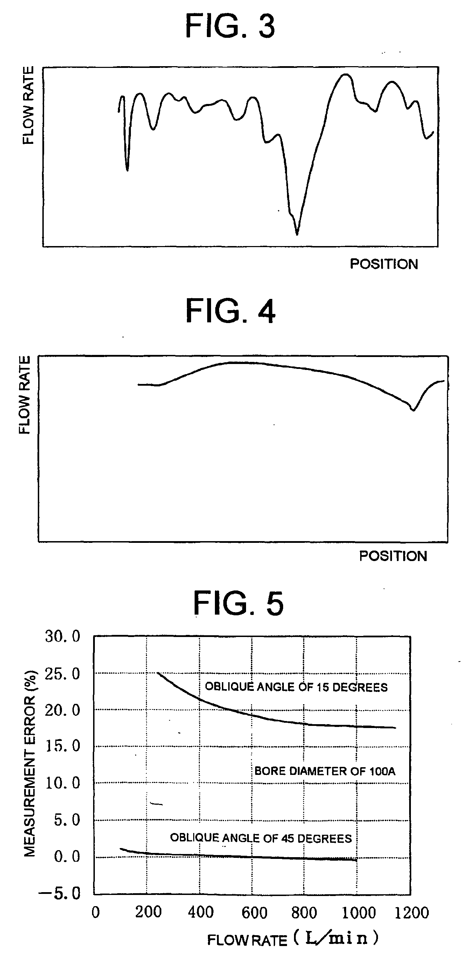

Figure 3      shows an example in which the flow rate to the position along the diameter direction of the piping is measured when the oblique angle of the ultrasound oscillator 51 is 15°.

Figure 4    shows an example in which the flow rate to the position along the diameter direction of the piping is measured when the oblique angle of the ultrasound oscillator 51 is 45°.

Figure 5    shows a comparison, with regard to the measurement errors of the flow rate output, between the first embodiment of the present invention and a conventional technique.

Figure 6    shows the structure of the conventional technique.

Figure 7    illustrates the principle of operation of a Doppler ultrasound flow meter.

Figure 8    shows the propagation status of an acoustic wave when the acoustic wave propagates in different mediums.

Figure 9    shows the measurement conditions shown in [3] and [4].

Figure 10    shows the relationship between the incidence angle and the transmission rate and the reflectivity as known from [5].

Figure 11    shows the relationship between the incidence angle and the transmission rate and the reflectivity as known from [5].

Figure 12    shows the behavior of the ultrasound echo in Figure 9.

Figure 13    shows a further enlarged view of Figure 9.

Figure 14    shows the flow rate profile for explaining the objective of the present invention.

Figure 1 shows the main part of a first embodiment. Although the structure shown in Figure 1 is substantially the same as that shown in Figure 9, Figure 12, Figure 13, etc., different reference numerals are used, for clarity.

In Figure 1, reference numeral 51 denotes an ultrasound oscillator, i.e., an ultrasound transducer, which generates an acoustic wave. This ultrasound oscillator 51 is made of a piezoelectric material, such as PZT (e.g., zircon, lead titanate) and operates both as an ultrasound transmitter and an ultrasound receiver. Reference numeral 52 denotes a wedge made of a resin material in which an acoustic wave can propagate (e.g., acrylic, epoxy resin, polyvinyl chloride, polyphenylene sulfide). The wedge 52 has an inclined plane 52a at the upper end thereof, and the ultrasound oscillator 51 is fixed to the inclined plane 52a by an epoxy adhesive agent or the like. The inclined plane 52a is inclined such that the oblique angle of the ultrasound oscillator 51 to the direction perpendicular to the longitudinal direction of the piping 53 (incidence angle of the ultrasound pulse at the interface between the wedge 52 and the piping 53) is equal to $\theta_{in}$. Reference numeral 54 denotes a fluid whose flow rate is to be measured.

The following section will describe a case with regards to the structure of Figure 1 in which the velocity of sound in the piping 53 is higher than that in the wedge 52, i.e., the wedge 52 is made of acrylic while the piping 53 is made of aluminum for example, and the fluid 54 is water.

The speed of sound in acrylic is about 2,730 m/s, the velocity of the longitudinal wave in aluminum is about 6,420 m/s while the velocity of the shear wave therein is about 3,040 m/s, and the speed of sound in water is about 1,500 m/s.

The piping 53 may be made of aluminum or other metal in which an acoustic wave can propagate (e.g., iron, steel, ductile cast iron, stainless steel, copper, lead, brass).

In Figure 1, there is a critical angle when an ultrasound pulse is transmitted from the wedge 52 into the piping 53 and when an ultrasound pulse is transmitted from the fluid 54 into the piping 53. As is clear from Snell's law, the critical angle of any material has the relation as shown in mathematical expression 5:

$$\frac{\sin\theta_{in}}{c_w} = \frac{\sin\theta_{pl}}{c_{pl}} = \frac{\sin\theta_{ps}}{c_{ps}} = \frac{\sin\theta_f}{c_f}$$

where

$c_w$ represents the acoustic velocity in the wedge 52,
$c_{pl}$ represents the acoustic velocity of the longitudinal wave in the piping 53,
$c_{ps}$ represents the acoustic velocity of the shear wave in the piping 53,
$c_f$ represents the acoustic velocity in the fluid 54,
$\theta_{in}$ represents the oblique angle of the acoustic wave in the wedge 52 (incidence angle to piping 53),
$\theta_{pl}$ represents the angle of the longitudinal wave in the piping 53 (refraction angle),
$\theta_{ps}$ represents the angle of the shear wave in the piping 53 (refraction angle), and
$\theta_f$ represents the incidence angle θ in the fluid 54.

When the respective members are provided as in the case described above in which the wedge 52 is made of acrylic, the piping 53 is made of aluminum, and the fluid 54 is made of water, then the critical angle of the longitudinal wave is 25.2° while the critical angle of the shear wave is 63.9° when ultrasound is transmitted from the wedge 52 into the piping 53. Thus, when the oblique angle $\theta_{in}$ of the ultrasound oscillator 51 (incidence angle at the interface between the wedge 52 and the piping 53) is within the above critical angle range (i. e., $25.2° \leq \theta_{in} \leq 63.9°$), only the shear wave propagates in the piping 53 because the longitudinal wave is totally reflected at the interface between the wedge 52 and the piping 53.

As a result, only the ultrasound along one measurement line caused by the shear wave in the piping 53 is transmitted into water and thus the ultrasound echo from the reflectors in water is also caused by only the shear wave. Specifically, the ultrasound oscillator 51 does not receive an ultrasound echo caused by a longitudinal wave, thus reducing the acoustic noise included in the flow rate to be measured. This improves the measurement accuracy of the flow rate profile and enables the flow rate to be calculated with a higher accuracy.

Next, an example will be specifically described in which the wedge 52 shown in Figure 1 produces an acoustic wave of an incidence angle $\theta_{in}$ of 45°.

When the acoustic wave propagates from the wedge 52 into the aluminum piping 53, the above incidence angle $\theta_{in}$ exceeds 25.2° (which is the critical angle of the longitudinal wave) and thus the longitudinal wave is totally reflected at the interface between the wedge 52 and the piping 53 and does not propagate in the piping 53. On the other hand, the shear wave propagates in the piping 53 with the refraction angle of 51.9°.

Next, when the acoustic wave is transmitted from the piping 53 into the fluid 54 (which is water), then only a longitudinal wave exits into the water. As a result, the longitudinal wave propagates in water at a refraction angle ($\theta_{fs}$ in Figure 1) of 22.8° along one measurement line.

The longitudinal wave reflected by the reflectors, i.e., the ultrasound echo, is also transmitted into the piping 53 at an incidence angle of 22.8°.

With regard to the transmission of the acoustic wave from water into the aluminum piping, data is shown in Figure 2, as provided in document [6]: "Acoustic Wave" (Cordon S. Kino).

Figure 2 shows the relation between the incidence angle of an ultrasound wave from water into the piping and the energy transmission coefficient (transmission rate) of the longitudinal wave and the shear wave in the piping.

According to Figure 2, the incidence angle to the piping 53 of 22.8° is equal to or higher than the critical angle of the longitudinal wave, and thus, the longitudinal wave is totally reflected at the interface between water and the piping 53. Specifically, the longitudinal wave does not propagate in the piping 53. Thus, the piping 53 has therein only one measurement line of the ultrasound echo produced by the shear wave and the ultrasound oscillator 51 receives the ultrasound echo of this shear wave, thus reducing the conventional acoustic noise caused by the longitudinal wave.

As described above, the measurement accuracy of the flow rate profile can be improved over conventional cases by improving the oblique angle of the ultrasound oscillator 51 (incidence angle to the piping 53) to eliminate the longitudinal wave in the piping 53.

Figure 3 and Figure 4 show examples in which the flow rate at a position along the radial direction of the piping 53 is measured when the oblique angle of the ultrasound oscillator 51 is 15° (Figure 3) and when the oblique angle of the ultrasound oscillator 51 is 45° (Figure 4).

When the oblique angle of the ultrasound oscillator 51 is 45° that is equal to or higher than the critical angle of the longitudinal wave when the ultrasound is transmitted from the wedge 52 into the piping 53 (25.2°) and that is equal to or lower than the critical angle of the shear wave (63.9°), then appropriate measurement values as shown in Figure 4 are obtained according to which the flow rate changes continuously depending on the position along the radial direction (along a diameter).

When the oblique angle of Figure 3 is 15°, the piping 53 has therein the longitudinal wave and the shear wave and thus the ultrasound echo received by the ultrasound oscillator 51 includes a large amount of acoustic noise, causing the measurement values of flow rate profile to be unstable, which deteriorates the measurement accuracy.

Figure 5 shows the comparison, with regard to the measurement errors that occurred when the flow rate output of an electromagnetic flow meter was measured based on the flow rate profile, between a case in which the oblique angle of the ultrasound oscillator 51 is similarly 45° according to this embodiment, and a case in which the oblique angle of the ultrasound oscillator 51 is 15°, as in conventional cases.

As can be seen from Figure 5, this embodiment also significantly improves the measurement errors when compared to conventional cases.

In a second embodiment of the present invention, only the longitudinal wave component of the ultrasound echo propagating in the piping 53 after being reflected by the reflectors in the fluid 54 is eliminated.

It is assumed that, when the fluid 54 is water for example, the critical angle of the longitudinal wave in the ultrasound echo transmitted into the aluminum piping 53 after being reflected by the reflectors in water is 13.5° while the critical angle of the shear wave is 29.6° when the acoustic velocity in water is 1500[m/s].

Thus, when the acoustic wave from the piping 53 into water has an incidence angle that is equal to or higher than 13.5° and that is equal to or lower than 29.6°, then only the shear wave element is transmitted into the piping 53 and the longitudinal wave element is eliminated when the ultrasound echo is transmitted from water into the piping 53, thus reducing the acoustic noise caused by the longitudinal wave.

As a result, the ultrasound oscillator 51 receives only the ultrasound echo of the shear wave in the piping 53, and this allows the piping 53 to carry reduced acoustic noise caused by the longitudinal wave, provides the measurement of a flow rate profile with a higher accuracy, and improves the accuracy of the measurement of a flow meter.

The wedge may be made of a metal in which an acoustic wave can propagate (e.g., iron, steel, cast iron, stainless steel, copper, lead, aluminum, brass), and the piping may be made of a resin in which an acoustic wave can propagate (e.g., polyvinyl chloride, acrylic, FRP, polyethylene, Teflon (registered trademark), tar epoxy, mortar).

As described above, one embodiment of the present invention of may be defined as a clamp-on type acoustic Doppler current profiler, characterized in that ultrasound is transmitted from an ultrasound transducer provided exterior to a tube body to a to-be-measured fluid within the tube body, to measure the flow rate profile of the to-be-measured fluid by using a mechanism whereby the frequency of ultrasound is changed by the Doppler effect when ultrasound is reflected by reflectors existing in the to-be-measured fluid,

wherein a wedge through which an acoustic wave can propagate is provided between a longitudinal ultrasound wave-generating source of the ultrasound transducer and the tube body, and

the source for generating an acoustic wave is fixed to the wedge at an inclination such that, when the velocities of the longitudinal wave and the shear wave of the ultrasound propagating in the tube body are equal to or higher than the velocity of the longitudinal wave in the wedge, the incidence angle of the ultrasound transmitted from the wedge into the tube body is equal to or higher than the critical angle of the longitudinal wave that is determined by the velocity of the longitudinal wave in the wedge and the velocity of the longitudinal wave in the tube body and is equal to or lower than the critical angle of the shear wave that is determined by the velocity of the longitudinal wave in the wedge and the velocity of the shear wave in the tube body.

Another embodiment of the present invention of may be defined as a clamp-on type acoustic Doppler current profiler, comprising:

an ultrasound transducer provided exterior to a tube body to a to-be-measured fluid in the tube body, to measure the flow rate profile of the to-be-measured fluid by using a mechanism in which the frequency of ultrasound is changed by Doppler effect when the ultrasound is reflected by reflectors existing in the to-be-measured fluid,

wherein a wedge through which an acoustic wave can propagate is provided between a longitudinal ultrasound wave-generating source of the ultrasound transducer and the tube body, and

the source for generating an acoustic wave is fixed to the wedge at an inclined angle such that, when the velocities of the longitudinal wave and the shear wave of the ultrasound propagating in the tube body are equal to or higher than the acoustic velocity in the to-be-measured fluid, the incidence angle of the ultrasound transmitted from the tube body into the to-be-measured fluid is equal to or higher than the critical angle of the longitudinal wave that is determined by the velocity in the to-be-measured fluid and the acoustic velocity of the longitudinal wave in the tube body and is equal to or lower than the critical angle of the shear wave that is determined by the velocity in the to-be-measured fluid and the acoustic velocity of the shear wave in the tube body.

**Claims**

1. A clamp-on type acoustic Doppler current profiler, comprising:

ultrasound transducer (51) provided exterior to a tube body (53) for transmitting ultrasound to a fluid (54) within the tube body (53) and for receiving an ultrasound echo reflected by reflectors existing in the fluid (54), and
means (10) for measuring the flow rate profile of the fluid (54) using the frequency difference between the transmitted and the reflected ultrasound caused by the Doppler effect,

**characterized in that**
a wedge (52) through which an acoustic wave can propagate is provided between a longitudinal ultrasound wave-generating source of the ultrasound transducer (51) and the tube body (53), and
the wave-generating source is fixed to the wedge (52) at an inclination such that, when the velocity of the longitudinal wave and that of the shear wave of the ultrasound propagating in the tube body (53) are equal to or higher than the velocity of the longitudinal wave in the wedge (52), the incidence angle of the ultrasound transmitted from the wedge (52) into the tube body (53) is equal to or higher than the critical angle of the longitudinal wave that is determined by the velocity of the longitudinal wave in the wedge (52) and the velocity of the longitudinal wave in the tube body (53) and is equal to or lower than the critical angle of the shear wave that is determined by the velocity of the longitudinal wave in the wedge (52) and the velocity of the shear wave in the tube body (53).

2. A clamp-on type acoustic Doppler current profiler, comprising:

ultrasound transducer (51) provided exterior to a tube body (53) for transmitting ultrasound to a fluid (54) within the tube body (53) and for receiving an ultrasound echo reflected by reflectors existing in the fluid (54), and
means (10) for measuring the flow rate profile of the fluid (54) using the frequency difference between the transmitted and the reflected ultrasound caused by the Doppler effect,

**characterized in that**
a wedge (52) through which an acoustic wave can propagate is provided between a longitudinal ultrasound wave-generating source of the ultrasound transducer (51) and the tube body (53), and
the wave-generating source is fixed to the wedge (52) at an inclination such that, when the velocity of the longitudinal wave and that of the shear wave of the ultrasound propagating in the tube body (53) are equal to or higher than the acoustic velocity in the fluid (54), the incidence angle of the ultrasound transmitted from the tube body (53) into the fluid (54) is equal to or higher than the critical angle of the longitudinal wave that is determined by the acoustic velocity in the fluid (54) and the velocity of the longitudinal wave in the tube body (53) and is equal to or lower than the critical angle of the shear wave that is determined by the acoustic velocity in the fluid (54) and the velocity of the shear wave in the tube body (53).

3. A clamp-on type acoustic Doppler current profiler according to Claim 1 or 2, **characterized in that** the wedge (52) is made of a resin in which an acoustic wave can propagate, such as acrylic, epoxy resin, polyvinyl chloride, and polyphenylene sulfide.

4. A clamp-on type acoustic Doppler current profiler according to any one of Claims 1 to 3, **characterized in that** the tube body (53) is made of a metal in which an acoustic wave can propagate, such as iron, steel, ductile cast iron, cast iron, stainless, copper, lead, aluminum, and brass.

5. A clamp-on type acoustic Doppler current profiler according to any one of Claims 1 to 3, **characterized in that** the tube body (53) is made of a resin in which an acoustic wave can propagate, such as polyvinyl chloride, acrylic, FRP, polyethylene, polytetrafluoroethylene, tar epoxy, and mortar.

6. A clamp-on type acoustic Doppler current profiler according to Claim 1, 2, 4, or 5, **characterized in that** the wedge (52) is made of a metal in which an acoustic wave can propagate, such as, iron, steel, cast iron, stainless, copper, lead, aluminum, and brass.

# FIG. 1

LATERAL LONGITU-
WAVE DINAL WAVE

# FIG. 2

CRITICAL ANGLE OF LONGITUDINAL WAVE

TRANSMITTED LATERAL WAVE

TRANSMISSION RATE OF
LONGITUDINAL WAVE
IN ALUMINUM PIPING

TRANSMISSION RATE OF
LATERAL WAVE IN
ALUMINUM PIPING

INCIDENCE ANGLE [DEGREES]

22.8 DEGREES

# FIG. 3

FLOW RATE

POSITION

# FIG. 4

FLOW RATE

POSITION

# FIG. 5

MEASUREMENT ERROR (%)

30.0
25.0
20.0
15.0
10.0
5.0
0.0
−5.0

OBLIQUE ANGLE OF 15 DEGREES

BORE DIAMETER OF 100A

OBLIQUE ANGLE OF 45 DEGREES

0    200    400    600    800    1000    1200

FLOW RATE ( L/min )

# FIG. 6

# FIG. 7

(A)

(B)

$$\frac{1}{F_{rpf}}$$

(C)

FLOW RATE

POSITION ALONG DIAMETER DIRECTION

# FIG. 8

$\theta_{in}$

MEDIUM 1

MEDIUM 2

$\theta_{out}$

# FIG. 9

# FIG. 10

# FIG. 11

# FIG. 12

# FIG .13

# FIG. 14